# EUROPÄISCHE PATENTSCHRIFT

(11) **EP 2 663 393 B1**
(45) Veröffentlichungstag und Bekanntmachung des Hinweises auf die Patenterteilung: **04.03.2015**
(21) Anmeldenummer: 12700948.8
(22) Anmeldetag: 09.01.2012
(51) Int. Cl.: B01F 17/54, A61K 8/06, A61K 8/55, A61K 8/892, A61Q 5/00, B01F 17/14, B01F 17/42, B01F 17/44

(54) **SILICONEMULSIONEN UND VERFAHREN ZU DEREN HERSTELLUNG**
SILICONE EMULSIONS AND METHOD FOR PRODUCING SAME
ÉMULSIONS DE SILICONE ET LEURS PROCÉDÉS DE PRÉPARATION

(30) Priorität: 13.01.2011 DE 102011002668
(43) Veröffentlichungstag der Anmeldung: 20.11.2013
(73) Patentinhaber: Wacker Chemie AG, 81737 München (DE)
(72) Erfinder: RAUTSCHEK, Holger, 01612 Nünchritz (DE); BEER, Gerhard, 84489 Burghausen (DE)
(74) Vertreter: Budczinski, Angelika
(86) Internationale Anmeldenummer: PCT/EP2012/050202
(87) Internationale Veröffentlichungsnummer: WO 2012/095374

(56) Entgegenhaltungen:
- WO-A1-2006/015740
- WO-A1-2011/032824
- DE-A1- 2 014 174

## Beschreibung

Die Erfindung betrifft wässrige Siliconemulsionen, die hochviskose Polyorganosiloxane enthalten und einen besonders niedrigen Gehalt an cyclischen Siloxanen aufweisen, Verfahren zu deren Herstellung sowie deren Verwendung.

Silicone sind vielfältig einsetzbar. Um die Anwendung und Dosierung insbesondere bei viskosen Produkten zu erleichtern, ist es für viele Anwendungen wünschenswert, dass die siliziumorganischen Verbindungen in verdünnter Form vorliegen. Die Verwendung von organischen Lösemitteln, wie Benzol oder Chlorkohlenwasserstoffen, für diesen Zweck ist zwar möglich, jedoch aus ökologischer und arbeitsmedizinischer Sicht nachteilig. Deshalb erfolgt der Einsatz meist in Form von wässrigen Emulsionen oder Dispersionen, üblicherweise als Öl-in-Wasser-Emulsionen (O/W-Emulsionen), die mit Wasser verdünnbar sind. Als Ölphase werden dabei die mit Wasser nicht mischbaren siliziumorganischen Verbindungen verstanden, gegebenenfalls gelöst in organischen Lösemitteln.

Für viele Anwendungen ist es vorteilhaft, wenn das Silicon ein hohes Molekulargewicht und somit eine hohe Viskosität hat. Ein bekannter Weg zu Emulsionen zu gelangen, die ein hochmolekulares Silicon enthalten, ist die Emulsionspolymerisation von niedermolekularen, insbesondere cyclischen Organosiloxanen mit A-rylalkylsulfonsäuren (DE-OS 14 95 512). Dabei werden durch intensives Rühren oder Homogenisieren mit einem Hochdruckhomogenisator außergewöhnlich niedrige Teilchengrößen erreicht, die mit einem optischen Mikroskop nicht mehr erkennbar sind. Nachteilig bei diesem Verfahren ist die Tatsache, dass aufgrund des Gleichgewichtscharakters dieser Reaktion bezogen auf Siloxan über 10% flüchtige cyclische Siloxane enthalten sind, die jedoch unerwünscht sind. Deshalb ist vorgeschlagen worden, diese nachträglich abzudestillieren (z.B. US-A 4,600,436) oder mit einem Membranverfahren zu entfernen (EP-A 1 368 109). Beide Verfahren bedeuten zusätzlichen technischen Aufwand und können die Stabilität der Emulsion beeinträchtigen.

Alternativ können anstelle cyclischer Siloxane lineare Oligomere mit endständigen Silanolgruppen verwendet werden. Aus diesen Oligomeren wird in Gegenwart von Emulgatoren, Kondensationskatalysatoren und einer sehr kleinen Wassermenge eine Paste gebildet, in der die Polykondensation stattfindet. Anschließend wird diese Paste auf die gewünschte Konzentration verdünnt (EP 93 310 B2). Im Allgemeinen sind die Anteile an cyclischen flüchtigen Siloxanen geringer als bei der Emulsionspolymerisation von cyclischen Siloxanen. Eine Verminderung des Anteils an diesen flüchtigen Siloxanen kann z.B. dadurch erfolgen, dass erst eine Emulsion aus der Salzform des anionischen Emulgators/Katalysators hergestellt wird, diese dann durch Zugabe von Säure aktiviert wird (EP-A 1 072 629). Das erhöht letztlich den Salzanteil in der Emulsion, was für die Stabilität nachteilig ist. Bei der Verwendung von alkoxyterminierten Siloxanoligomeren sollen ebenfalls weniger Cyclen gebildet werden (JP-A2001288269). Allerdings sind diese Oligomere aufwändiger herzustellen und damit kostenintensiver.

Spezielle Emulgatoren auf der Basis von Taurocholaten tragen ebenfalls zur Reduzierung der Menge an Cyclen bei, die bei der Emulsionskondensation von Siloxanoligomeren gebildet werden (WO 2006102010). Aber auch hier werden, wie die Ausführungsbeispiele deutlich zeigen, über 1% Octamethylcyclotetrasiloxan gebildet.

Es ist auch vorgeschlagen worden, Dimethylpolysiloxane, insbesondere Polysiloxane, die mit Trimethylsiloxygruppen terminiert sind, mit Viskositäten von bis zu 5000000 cSt zu emulgieren, in dem diese mit 10-30% bezogen auf Siloxan eines Phosphorsäurepartialesters vermischt und erhitzt werden, bis eine klare Lösung entsteht, die nach Neutralisation mit Wasser verdünnt wird (DE-A 27 30 923). Allerdings hat dieses Verfahren den Nachteil, dass das Polydimethylsiloxan dabei meist depolymerisiert wird, so dass die erhaltene Emulsion ein niedrig viskoses Siloxan und einen hohen Anteil an flüchtigen cyclischen Siloxanen z.B. Octamethylcyclotetrasiloxan enthält. Weiter Siliconemulsionen sind aus WO 2011/032824 A1 bekannt.

In JP2002020490 wird vorgeschlagen, als Emulgatoren mindestens eine Zweierkombination von Polyoxyethylenalkylsulfaten, Polyoxyethylenalkylphosphaten und Alkylsulfonaten bzw. die entsprechenden Säuren einzusetzen, wobei bevorzugt die Säure erst in der Emulsion durch Zusatz von Mineralsäuren wie Schwefelsäure frei gesetzt wird. Eine alleinige Verwendung von Polyoxyethylenalkylphosphaten soll nur zu niedermolekularen Polyorganosiloxanen führen, da deren katalytische Aktivität zu niedrig ist. Deshalb sind Kombinationen mit Sulfaten oder Sulfonaten und eine Aktivierung von Schwefelsäure notwendig. Das führt letztlich wiederum zu mehr als 1% cyclischer Siloxanoligomere, es sei denn die Reaktionszeit ist extrem kurz, wobei jedoch keine Viskositäten > 1 m²/s (1000000 mm²/s) erreicht werden.

Auf der anderen Seite werden derartige Emulsionen praktisch oft in der Art und Weise hergestellt, dass entweder mehrere Chargen diskontinuierlich hergestellt und in einen Reifetank transferiert werden oder eine kontinuierliche Kampagne über einen bestimmten Zeitraum in einen Reifetank produziert wird, wo dann nach Erreichen der gewünschten Viskosität die Reaktion durch Neutralisation abgebrochen wird. Dabei ist es unvermeidlich dass ein nicht geringer Teil der Emulsion länger als erforderlich im Tank verweilt, wodurch der Anteil an cyclischen Oligomeren das tolerable Maß übersteigt.

Gegenstand der Erfindung sind Emulsionen von Polyorganosiloxanen enthaltend
(A) Polyorganosiloxane, die eine Viskosität größer als 10 000 mm²/s, gemessen bei 25°C, aufweisen,
(B) mindestens einen Emulgator der Formel

   (RO)ₙP(O)(OH)₍₃₋ₙ₎ (I),

   worin
   R gleich oder verschieden sein kann und einwertige Kohlenwasserstoffreste mit 4 bis 30 Kohlenstoffatomen bedeutet n 1 oder 2 ist,
   und/oder dessen Salze,
(C) mindestens einen Emulgator ausgewählt aus der Gruppe bestehend aus
(C1) ethoxylierten Triglyceriden mit 40 bis 400 Ethylenglycolgruppen,
(C2) ethoxylierten Sorbitanestern von Fettsäuren mit 12 bis 18 Kohlenstoffatomen und 10 bis 40 Ethylenglycolgruppen,
(C3) Verbindungen der Formel

   R¹-O-(CH₂CH₂O)ₘ-H (II)

   und
(C4) Verbindungen der Formel

   R²CH₂C(O)-O-(CH₂CH₂O)ₚ-H (III),

   worin
   R¹ einen linearen Alkylrest mit 10 bis 30 Kohlenstoffatomen bedeutet,
   R² einen Alkylrest mit 10 bis 30 Kohlenstoffatomen bedeutet,
   m einen Wert von 15 bis 100 darstellt und
   p einen Wert von 15 bis 100 annimmt,
   sowie
(D) Wasser,
   mit der Maßgabe, dass die Emulsionen weniger als 2 Gew.-% Octaorganylcyclotetrasiloxan (D₄), bezogen auf Komponente (A), enthalten.

Die erfindungsgemäßen Emulsionen können nach dem Fachmann bekannten Verfahren hergestellt werden.

Ein weiterer Gegenstand der Erfindung ist ein Verfahren zur Herstellung der erfindungsgemäßen Emulsionen, dadurch gekennzeichnet, dass
(a) Polyorganosiloxane enthaltend Einheiten der allgemeinen Formel

   R⁴ₐ(R³O)_{b}SiO_{(4-a-b)/2} (IV),

   worin
   R⁴ gleich oder verschieden sein kann und einen einwertigen, gegebenenfalls substituierten Kohlenwasserstoffrest mit 1 bis 30 Kohlenstoffatomen oder Wasserstoffatom bedeutet,
   R³ gleich oder verschieden sein kann und Wasserstoffatom oder einen einwertigen, gegebenenfalls substituierten Kohlenwasserstoffrest bedeutet,
   a 0, 1, 2 oder 3 ist und
   b 0, 1, 2 oder 3 ist,
   mit der Maßgabe, dass die Summe a+b kleiner oder gleich 3 ist und die Polyorganosiloxane 5 bis 500 Einheiten der Formel (IV) enthalten, wobei in mindestens einer Einheit b verschieden 0 ist,
(b) Emulgator der Formel (I), dessen OH-Gruppen gegebenenfalls teilweise neutralisiert sein können,
(c) Emulgator ausgewählt aus der Gruppe bestehend aus ethoxylierten Triglyceriden mit 40 bis 400 Ethylenglycolgruppen, ethoxylierten Sorbitanestern von Fettsäuren mit 12 bis 18 Kohlenstoffatomen und 10 bis 40 Ethylenglycolgruppen, Verbindungen der Formel (II) und Verbindungen der Formel (III),
(d) Wasser und
   gegebenenfalls
(e) weitere Stoffe
   durch Rühren und/oder Homogenisieren vermischt werden sowie die Organopolysiloxane (a) enthaltend Einheiten der Formel (IV) bei Temperaturen von 0 bis 50°C kondensieren gelassen werden bis die gewünschte Viskosität erreicht ist und gegebenenfalls anschließend der Emulgator der Formel (I) mit Basen neutralisiert wird, so dass der pH-Wert der Emulsion größer als 5 ist, und gegebenenfalls weiteres Wasser (d) und/oder weitere Stoffe (e) zugegeben werden.

Als Misch- und Homogenisierwerkzeug können alle dem Fachmann bekannten Emulgiergeräte, wie beispielsweise schnelllaufende Rührer, Dissolverscheiben, Rotor-Stator-Homogenisatoren, Ultraschallhomogenisatoren und Hochdruckhomogenisatoren verschiedenster Bauart, verwendet werden.

Das erfindungsgemäße Verfahren kann kontinuierlich, semikontinuierlich oder diskontinuierlich betrieben werden.

Eine bevorzugte Ausführungsform des erfindungsgemäßen Verfahrens ist dadurch gekennzeichnet, dass
in einem 1. Schritt
(a) 100 Gewichtsteile Polyorganosiloxane enthaltend Einheiten der allgemeinen Formel

   R⁴ₐ(R³O)_{b}SiO_{(4-a-b)/2} (IV),

   worin
   R⁴ gleich oder verschieden sein kann und einen einwertigen, gegebenenfalls substituierten Kohlenwasserstoffrest mit 1 bis 30 Kohlenstoffatomen oder Wasserstoffatom bedeutet,
   R³ gleich oder verschieden sein kann und Wasserstoffatom oder einen einwertigen, gegebenenfalls substituierten Kohlenwasserstoffrest bedeutet,
   a 0, 1, 2 oder 3 ist und
   b 0, 1, 2 oder 3 ist,
   mit der Maßgabe, dass die Summe a+b kleiner oder gleich 3 ist und die Organopolysiloxane 5 bis 500 Einheiten der Formel (IV) enthalten, wobei in mindestens einer Einheit b verschieden 0 ist,
(b) 1 bis 30 Gewichtsteile eines Emulgators der Formel (I), dessen OH-Gruppen gegebenenfalls teilweise neutralisiert sein können,
(c) 1 bis 30 Gewichtsteile eines Emulgators ausgewählt aus der Gruppe bestehend aus ethoxylierten Triglyceriden mit 40 bis 400 Ethylenglycolgruppen, ethoxylierten Sorbitanestern von Fettsäuren mit 12 bis 18 Kohlenstoffatomen und 10 bis 40 Ethylenglycolgruppen, Verbindungen der Formel (II) und Verbindungen der Formel (III),
(d) 1 bis 50 Gewichtsteile Wasser und
   gegebenenfalls
(e) weitere Stoffe
   durch Rühren und/oder Homogenisieren vermischt werden,
   in einem gegebenenfalls durchgeführten 2. Schritt
   weiteres Wasser (d) zugegeben wird,
   in einem 3. Schritt
   die Organopolysiloxane (a) enthaltend Einheiten der Formel (IV) bei Temperaturen von 0 bis 50°C kondensieren gelassen werden bis die gewünschte Viskosität erreicht ist,
   in einem gegebenenfalls durchgeführten 4. Schritt
   der Emulgator der Formel (I) mit Basen neutralisiert wird, so dass der pH-Wert der Emulsion größer als 5 ist und
   in einem gegebenenfalls durchgeführten 5. Schritt
   die im 4. Schritt erhaltene Emulsion mit weiterem Wasser (d) und/oder weiteren Stoffen (e) vermischt wird.

Bei den Polyorganosiloxanen (A), welche in den erfindungsgemäßen Emulsionen enthalten sind, handelt es sich vorzugsweise um solche enthaltend Einheiten der Formel (IV), besonders bevorzugt um solche aus Einheiten der Formel (IV) mit einem durchschnittlichen Wert von a von 1,990 bis 2,005 und einem durchschnittlichen Wert von b von 0,001 bis 0,004, insbesondere um solche aus Einheiten der Formel (IV) mit R³ gleich Wasserstoffatom, R⁴ gleich Methylrest und einem durchschnittlichen Wert von a von 1,990 bis 2,005 und einem durchschnittlichen Wert von b von 0,001 bis 0,004. Ganz besonders bevorzugt handelt es sich bei den Polyorganosiloxanen (A) um Dimethylpolysiloxane, die Trimethylsiloxy- und/oder Dimethylhydroxysiloxyendgruppen tragen.

Polyorganosiloxane (A), welche in den erfindungsgemäßen Emulsionen enthalten sind, haben eine Viskosität von vorzugsweise größer als 0.1 m²/s (100 000 mm²/s,) besonders bevorzugt größer als 1 m²/s 1 000 000 mm²/s, jeweils bei 25°C.

Beispiele für Reste R sind verzweigte oder unverzweigte Alkylreste mit 4 bis 30 Kohlenstoffatomen, wie Butyl-, Hexyl-, 2-Ethylhexyl-, Octyl-, Isononyl-, n-Decyl-, Dodecyl-, Isotridecyl- und n-Tetradecylreste, ungesättigte aliphatische Reste, wie Oleylreste, sowie aromatische Reste, wie Phenyl-, Toloyl-, Xylyl-, Nonylphenyl-, Naphthyl-, Anthracyl-, Tristyrylphenyl- oder Benzylreste.

Bevorzugt handelt es sich bei Rest R um Alkylreste mit 4 bis 18 Kohlenstoffatomen, besonders bevorzugt um n-Butyl-, n-Octyl-, 2-Ethylhexyl-, n-Decyl-, n-Dodecyl- oder n-Tetradecylreste, insbesondere um n-Octyl- und n-Decylreste.

Beispiele für erfindungsgemäß eingesetzte Verbindungen der Formel (I) sind Di-n-butylphosphat, Di-n-hexylphosphat, Mono-n-octylphosphat, Di-n-octylphosphat, Mono-2-ethylhexylphosphat, Di-2-ethylhexylphosphat, Mono-i-nonylphosphat, Di-i-nonylphosphat, Mono-n-decylphosphat, n-Octyl-n-Decylphosphat, Di-n-decylphosphat, Monoisotridecylphosphat, Di-n-nonylphenylphosphat, Monooleylphosphat und Distearylphosphat.

Bevorzugt handelt es sich bei den erfindungsgemäß eingesetzten Verbindungen der Formel (I) um Mono-n-octylphosphat, Di-n-octylphosphat, Mono-n-decylphosphat, n-Octyl-n-Decylphosphat und Di-n-decylphosphat.

Vorzugsweise handelt es sich bei den erfindungsgemäß eingesetzten Verbindungen der Formel (I) um Mischungen von Diestern und Monoestern.

Die erfindungsgemäßen Emulsionen können als Komponente (B) nun Verbindungen der Formel (I) als solche enthalten oder deren Salze, bevorzugt mit Alkali- bzw. Erdalkalihydroxiden, Ammoniak oder Aminen, oder Gemische aus Säuren der Formel (I) und deren Salze.

Bevorzugt handelt es sich bei Komponente (B) der erfindungsgemäßen Emulsionen um Salze der Verbindungen der allgemeinen Formel (I), insbesondere um Alkalisalze oder Triethanolaminsalze.

Die Säurezahl der in der erfindungsgemäßen Emulsion enthaltenen Komponente (B) wird durch deren Anzahl an freien OH-Gruppen sowie deren Molmasse bestimmt, also die Menge an KOH in mg, die zur Neutralisation von 1 g Komponente (B) benötigt wird. Die Säurezahl der Komponente (B) liegt vorzugsweise im Bereich von 0 bis 200, besonders bevorzugt im Bereich von 0 bis 20, insbesondere bei 0, d.h. als Komponente (B) enthalten die erfindungsgemäßen Emulsionen in diesem Fall vollständig neutralisierte Verbindungen der Formel (I).

Verbindungen der Formel (I) sind kommerziell verfügbar bzw. nach allgemein bekannten chemischen Methoden darstellbar.

Die erfindungsgemäßen Emulsionen enthalten als weiteren Emulgator (C) mindestens einen nichtionogenen Emulgator ausgewählt aus Verbindungen der Formeln (II) und (III), ethoxylierten Triglyceriden oder ethoxylierten Sorbitanestern.

Beispiele für Reste R¹ und R² sind unabhängig voneinander der n-Decyl-, n-Dodecyl-, n-Tetradecyl- n-Hexadecyl-, n-Octadecyl- und der n-Octadecenylrest.

Es handelt sich bein Rest R¹ um lineare Alkylreste mit 12 bis 20 Kohlenstoffatomen. Der Alkylrest R¹ weist insbesondere eine gerade Anzahl an Kohlenstoffatomen auf.

Bevorzugt handelt es sich bei Rest R² um Alkylreste mit 12 bis 20 Kohlenstoffatomen, besonders bevorzugt um lineare Alkylreste. Der Alkylrest R² weist insbesondere eine gerade Anzahl an Kohlenstoffatomen auf.

Bevorzugt hat m einen Wert von 20 bis 40.

Bevorzugt hat p einen Wert von 20 bis 50.

Beispiele für ethoxylierte Triglyceride mit 40 bis 400 Ethylenglycolgruppen (C1) sind
ethoxyliertes Rizinusöl mit 200 Ethylenglycoleinheiten,
ethoxyliertes Rizinusöl mit 40 Ethylenglycoleinheiten und
ethoxyliertes hydriertes Rizinusöl mit 200 Ethylenglycoleinheiten.

Beispiele für ethoxylierte Sorbitanester von Fettsäuren mit 12 bis 18 Kohlenstoffatomen und 10 bis 40 Ethylenglycolgruppen (C2) sind
Polyoxyethylen(20)Sorbitanstearat (Polysorbat 60),
Polyoxyethylen(20)Sorbitantristearat (Polysorbat 65),
Polyoxyethylen(20)Sorbitanoleat (Polysorbat 80) und
Polyoxyethylen(20)Sorbitanlaurat (Polysorbat 20).

Beispiele für Verbindungen (C3) der Formel (II) sind
C₁₈H₃₇-O-(CH₂CH₂O)₂₀-H,
C₁₈H₃₅-O-(CH₂CH₂O)₂₀-H und
C₁₂H₂₃-O-(CH₂CH₂O)₂₃-H.

Beispiele für Verbindungen (C4) der Formel (III) sind C₁₆H₃₃-CH₂-C(O)-O-(CH₂CH₂O)₂₀-H, C₁₆H₃₃-CH₂-C(O)-O-(CH₂CH₂O)₃₀-H, C₁₆H₃₃-CH₂-C(O)-O-(CH₂CH₂O)₄₀-H und C₁₆H₃₃-CH₂-C(O)-O-(CH₂CH₂O)₁₀₀-H. Vorzugsweise hat der in der erfindungsgemäßen Emulsion enthaltene Emulgator (C) einen HLB-Wert größer 14, besonders bevorzugt größer 15,5. insbesondere 16,5 bis 20. Der HLB-Wert ist Ausdruck des Gleichgewichts zwischen hydrophilen und hydrophoben Gruppen eines Emulgators. Die Definition des HLB-Werts sowie Verfahren zu deren Ermittlung sind dem Fachmann bekannt und z.B. in Journal of Colloid and Interface Science 298 (2006) 441-450 sowie der dort zitierten Literatur insbesondere Zitat [23] beschrieben.

Vorzugsweise handelt es sich bei Emulgator (C) um eine Verbindung der Formel (II).

Zusätzlich zu den Komponenten (A), (B), (C) und (D) können die erfindungsgemäßen Emulsionen alle weiteren Stoffe enthalten, die üblicherweise Siliconemulsionen zugesetzt werden, wie z.B. weitere Siloxane, die verschieden sind zu Komponente (a), Silane, insbesondere Alkoxysilane, weitere Emulgatoren, die verschieden sind zu Komponenten (b) und (c), Verdicker und/oder Schutzkolloide sowie Additive, wie beispielsweise Konservierungsmittel, Desinfektionsmittel, Netzmittel, Korrosionsinhibitoren, Farbstoffe und Duftstoffe.

Bei den erfindungsgemäßen Emulsionen handelt es sich bevorzugt um solche, die
Komponente (A) zu bevorzugt 1 bis 80 Gew.-%, besonders bevorzugt zu 20 bis 70 Gew.-%,
Komponente (B) zu bevorzugt 0,2 bis 20 Gew.-%, besonders bevorzugt zu 1 bis 10 Gew.-% und
Komponente (C) zu bevorzugt 0,2 bis 20 Gew.-%, besonders bevorzugt zu 1 bis 10 Gew.-%,
enthalten.

Die erfindungsgemäßen Emulsionen enthalten vorteilhafterweise keine bzw. einen sehr geringen Anteil an cyclischen Siloxanen, insbesondere an Octaorganylcyclotetrasiloxanen (D₄). Die Organylgruppen in den Cyclosiloxanen richten sich nach den Organylgruppen im eingesetzten Organopolysiloxan und sind bevorzugt Methylgruppen.

Die erfindungsgemäße Emulsion enthält bevorzugt weniger als 1 Gew.-%, besonders bevorzugt weniger als 0,5 Gew.-%, insbesondere weniger als 0,1 Gew.-%, Octaorganylcyclotetrasiloxan, insbesondere Octamethylcyclotetrasiloxan (D₄), jeweils bezogen auf Komponente (A).

Die erfindungsgemäße Emulsion hat einen Teilchendurchmesser von vorzugsweise 50 bis 1000 nm, besonders bevorzugt von 100 bis 500 nm, insbesondere von 100 bis 200 nm, wobei sich diese Angaben auf den Mittelwert der Volumenverteilung gemessen nach dem Prinzip der Fraunhoferbeugung (entsprechend ISO 13320) beziehen.

Die erfindungsgemäßen Emulsionen haben einen Gehalt an nichtflüchtigen Anteilen gemessen nach DIN EN ISO 3251 von bevorzugt 1 bis 80 Gew.-%, besonders bevorzugt von 10 bis 65 Gew.-%, insbesondere von 30 bis 60 Gew.-%.

Der pH-Wert der erfindungsgemäßen Emulsion beträgt vorzugsweise 5 bis 10, besonders bevorzugt 6 bis 8, insbesondere in etwa 7.

Beispiele für Kohlenwasserstoffreste R⁴ sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, 1-Propenyl- und der 2-Propenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der der α- und der β-Phenylethylrest.

Beispiele für substituierte Reste R⁴ sind mit Halogen-, Cyano-, Glycidoxy-, Polyalkylenglycol- oder Aminogruppen substituierte Reste, wie beispielsweise Trifluorpropyl-, Cyanoethyl-, Glycidoxypropyl-, Polyalkylenglycolpropyl-, Aminopropyl- oder Aminoethylaminopropylreste.

Bevorzugt hat in den Einheiten der Formel (IV) maximal 1 Rest R⁴ die Bedeutung von Wasserstoffatom.

Bevorzugt handelt es sich bei Rest R⁴ um Kohlenwasserstoffreste mit 1 bis 18 Kohlenstoffatomen, besonders bevorzugt um den Methyl- oder den Phenylrest, wobei insbesondere mehr als 80 Mol-% der Reste R⁴ im Siloxan (a) die Bedeutung von Methylreste haben.

Beispiele für Reste R³ sind die für Reste R⁴ angegebenen Beispiele.

Bevorzugt handelt es sich bei Rest R³ um Wasserstoffatom und Kohlenwasserstoffreste mit 1 bis 4 Kohlenstoffatomen, besonders bevorzugt um Wasserstoffatom.

In Formel (IV) hat die Summe a+b einen Wert von bevorzugt durchschnittlich 1,5 bis 2,4, besonders bevorzugt durchschnittlich 1,8 bits 2,3, insbesondere durchschnittlich 1,9 bis 2,1.

Die im erfindungsgemäßen Verfahren eingesetzten Siloxane (a) bestehen bevorzugt aus 5 bis 500, besonders bevorzugt aus 10 bis 200, insbesondere aus 20 bis 100, Einheiten der Formel (IV) .

In bevorzugt 0,4 bis 40 %, besonders bevorzugt 2 bis 10 %, der Einheiten der Formel (IV) der im erfindungsgemäßen Verfahren eingesetzten Siloxane (a) ist b ungleich 0.

Beispiele für erfindungsgemäß eingesetzte Siloxane (a) sind mit Alkoxy- oder Hydroxygruppen terminierte Polydiorganosiloxane, insbesondere Polydiethyl- und Polydimethylsiloxane.

Die im erfindungsgemäßen Verfahren eingesetzten Siloxane (a) haben eine Viskosität von bevorzugt 5 bis 10 000 mm²/s, besonders bevorzugt 10 bis 500 mm²/s, insbesondere 30 bis 100 mm²/s, jeweils bei 25°C.

Bevorzugt handelt es sich bei den Siloxanen (a) um solche der Formel

HO[SiR⁴₂O]_{c}-H (V),

wobei R⁴ eine der obengenannten Bedeutungen hat, insbesondere Methylrest, und c einen Wert von 5 bis 500, bevorzugt von 10 bis 200, besonders bevorzugt aus 20 bis 100, besitzt.

Die Polysiloxane (a) enthaltend Einheiten der Formel (IV) sind handelsübliche Produkte bzw. können nach bekannten Verfahren hergestellt werden.

Beispiele für Komponente (b) sind die oben genannten Beispiele für die Verbindungen der Formel (I) gegebenenfalls im Gemisch mit deren Salze.

Die Säurezahl der im erfindungsgemäßen Verfahren eingesetzten Verbindung der Formel (I) wird durch den durchschnittlichen Wert von n und ihrer Molmasse bestimmt, also die Menge an KOH in mg, die zur Neutralisation von 1 g Verbindung der Formel (I) benötigt wird. Die Säurezahl der erfindungsgemäß eingesetzten Verbindung der Formel (I) liegt vorzugsweise im Bereich von 100 bis 600, besonders bevorzugt im Bereich von 200 bis 500, insbesondere im Bereich von 250 bis 450.

Komponente (b) wird im erfindungsgemäßen Verfahren in Mengen von vorzugsweise 1 bis 25 Gewichtsteilen, insbesondere 2 bis 10 Gewichtsteilen, jeweils bezogen auf 100 Gewichtsteile Polyorganosiloxan (a), eingesetzt.

Beispiele für Komponente (c) sind die oben genannten Beispiele für die Verbindungen (C).

Komponente (c) wird im erfindungsgemäßen Verfahren in Mengen von vorzugsweise 1 bis 25 Gewichtsteilen, insbesondere 5 bis 20 Gewichtsteilen, jeweils bezogen auf 100 Gewichtsteile Polyorganosiloxan (a), eingesetzt.

Als Wasser (d) können alle Arten von Wässer eingesetzt werde, die auch bisher zur Herstellung von Dispersionen eingesetzt wurden.

Als Wasser (d) wird vorzugsweise teil- oder vollentsalztes Wasser, destilliertes oder (mehrfach) redestilliertes Wasser, Wässer für medizinische oder pharmazeutische Zwecke, wie z.B. gereinigtes Wasser (Aqua purificata gemäß Pharm. Eur.) eingesetzt.

Das erfindungsgemäß eingesetzte Wasser (d) hat vorzugsweise eine Leitfähigkeit von weniger als 50 µS/cm, besonders bevorzugt weniger als 10 µS/cm, insbesondere weniger als 1,3 µS/cm, jeweils bei 25°C und 1010 hPa.

Wasser (d) wird im erfindungsgemäßen Verfahren in Mengen von vorzugsweise 20 bis 1000 Gewichtsteilen, insbesondere 30 bis 400 Gewichtsteilen, jeweils bezogen auf 100 Gewichtsteile Polyorganosiloxan (a), eingesetzt. Bei der bevorzugten Verfahrensvariante wird Wasser im ersten Schritt, im gegebenenfalls durchgeführten zweiten Schritt und im gegebenenfalls durchgeführten fünften Schritt zugegeben, wobei im ersten Schritt des erfindungsgemäßen Verfahrens Wasser in Mengen von vorzugsweise 1 bis 30 Gewichtsteilen, insbesondere 5 bis 20 Gewichtsteilen, jeweils bezogen auf 100 Gewichtsteile Polyorganosiloxan (a), eingesetzt wird.

Zusätzlich zu den Komponenten (a), (b), (c) und (d) können im erfindungsgemäßen Verfahren nun alle weiteren Stoffe (e), die üblicherweise Siliconemulsionen zugesetzt werden, eingesetzt werden, wie z.B. weitere Siloxane, die verschieden sind zu Komponente (a), Silane, insbesondere Alkoxysilane, weitere Emulgatoren, die verschieden sind zu Komponenten (b) und (c), Verdicker und/oder Schutzkolloide sowie Additive, wie beispielsweise Konservierungsmittel, Desinfektionsmittel, Netzmittel, Korrosionsinhibitoren, Farbstoffe und Duftstoffe. Der Zusatz dieser Komponenten (e) kann im ersten Schritt des erfindungsgemäßen Verfahrens und/oder auch in einem späteren Verfahrensschritt, z.B. nach dem 5. Schritt, erfolgen.

Beispiele für weitere Siloxane (e), die erfindungsgemäß eingesetzt werden können, sind solche der Formel (IV) mit b gleich 0, wie z.B. trimethylsiloxyterminierte Polydimethylsiloxane. Solche Siloxane (e) werden vorteilhafterweise eingesetzt, um die nach der Kondensationsreaktion erhaltenen Viskosität des Polysiloxans in der Emulsion zu steuern.

Falls weitere Siloxane (e) eingesetzt werden, handelt es sich um Mengen von bevorzugt 0,01 bis 10 Gewichtsteilen, bezogen auf 100 Gewichtsteile Komponente (a). Im erfindungsgemäßen Verfahren werden bevorzugt keine weiteren Siloxane (e) eingesetzt.

Beispiele für Silane (e), die erfindungsgemäß eingesetzt werden können, sind Methyltrimethoxysilan, Tetraethoxysilan, Vinyltriethoxysilan, Morpholinomethyltriethoxysilan, Cyclohexylaminomethyl-methyldiethoxysilan, die in DE-A 102005022099, Abschnitt [0052] und die in DE-A 102004038148, Abschnitt [0033] genannten Beispiele oder deren Hydrolyse-/Kondensationsprodukte. Solche Silane (e) werden vorteilhafterweise eingesetzt, um verzweigte oder vernetzte Siloxane z.B. solche, die nach der Trocknung der Emulsion elastische Filme bilden, zu erhalten. Bei der bevorzugten Verfahrensvariante können diese Silane (e) im 1. Schritt wie auch nach dem 3. Schritt zugesetzt werden.

Falls Silane (e) eingesetzt werden, handelt es sich um Mengen von bevorzugt 0,01 bis 10 Gewichtsteilen, bezogen auf 100 Gewichtsteile Komponente (a). Im erfindungsgemäßen Verfahren werden bevorzugt keine Silane (e) eingesetzt.

Beispiele für weitere Emulgatoren (e), die erfindungsgemäß eingesetzt werden können, sind alle bisher bekannten Emulgatoren, wie anionische oder nichtionische Emulgatoren, wie beispielsweise Alkylsulfate, ethoxylierte Alkylsulfate, und Polyethylenglycolether von Alkylphenolen und Alkylpolyglycoside.

Im erfindungsgemäßen Verfahren werden vorzugsweise keine kationischen und keine amphoteren Emulgatoren eingesetzt.

Im erfindungsgemäßen Verfahren werden vorzugsweise keine weiteren anionischen Emulgatoren, insbesondere keine Alkyl- oder Alkylarylbenzolsulfonsäuren oder deren Salze, als Komponente (e) eingesetzt.

Falls weitere Emulgatoren (e) eingesetzt werden, handelt es sich um Mengen von bevorzugt 1 bis 20 Gewichtsteilen, bezogen auf 100 Gewichtsteile Komponente (a).

Vorzugsweise werden als Komponente (e) keine weiteren Emulgatoren eingesetzt.

Falls bei dem erfindungsgemäßen Verfahren als Komponente (e) Verdicker bzw. Schutzkolloide eingesetzt werden, handelt es sich bevorzugt um Acrylsäurecopolymere.

Falls Verdicker und/oder Schutzkolloide (e) eingesetzt werden, handelt es sich um Mengen von bevorzugt 0,01 bis 2 Gewichtsteilen, bezogen auf 100 Gewichtsteile Komponente (a). Im erfindungsgemäßen Verfahren wird bevorzugt kein Verdicker und/oder Schutzkolloid (e) eingesetzt.

Beispiele für Additive (e), die erfindungsgemäß eingesetzt werden können, sind z.B. dem Fachmann bekannte Konservierungsmittel, Farb- oder Duftstoffe, insbesondere Konservierungsmittel, wie Methylisothiazolinon, Chlormethylisothiazolinon, Benzylisothiazolinon, Phenoxyethanol, Methylparaben, Ethylparaben, Propylparaben, Butylparaben, Isobutylparaben, Alkalibenzoate, Alkalisorbate, Iodopropinylbutylcarbamat, Benzylalkohol und 2-Brom-2-nitropropan-1,3-diol.

Falls Additive (e) eingesetzt werden, handelt es sich um Mengen von bevorzugt 0,0005 bis 2 Gewichtsteilen, bezogen auf 100 Gewichtsteile Komponente (a). Im erfindungsgemäßen Verfahren werden bevorzugt Additive (e) eingesetzt.

Im ersten Schritt der bevorzugten Ausführungsform des erfindungsgemäßen Verfahrens können alle Komponenten durch Rühren und/oder Homogenisieren, z.B. in beliebiger Reihenfolge, miteinander vermischt werden, wobei die Umfangsgeschwindigkeit des Rührers und/oder Rotor-Stator Homogenisators bevorzugt größer als 5 m/s, besonders bevorzugt größer als 10 m/s ist, insbesondere 5 bis 50 m/s.

Verbindung der Formel (I) als Komponente (b) kann, falls erwünscht, bereits im ersten Schritt des erfindungsgemäßen Verfahrens teilweise mit Basen, wie z.B. Alkalihydroxiden oder Aminen, neutralisiert werden, was jedoch nicht bevorzugt ist.

Die Mischung gemäß erstem Schritt des erfindungsgemäßen Verfahrens hat einen pH-Wert von kleiner 6, vorzugsweise kleiner 5, besonders bevorzugt kleiner 4, insbesondere 1 bis 3.

Vorzugsweise ist die im ersten Schritt erhaltene Emulsion aus Komponenten (a), (b), (c), (d) und gegebenenfalls (e) hochviskos und nicht fließfähig. Besonders bevorzugt ist es, wenn die Fließgrenze (entsprechend DIN 53019-1 und zitierten Normen) der im ersten Schritt erhaltenen Emulsion größer als 100 Pa, insbesondere größer als 1000 Pa ist.

Der erste Schritt des erfindungsgemäßen Verfahrens wird bei Temperaturen von vorzugsweise 5 bis 80°C, insbesondere 10 bis 50°C, und dem Druck der umgebenden Atmosphäre, also zwischen 900 und 1100 hPa, oder bei einem erhöhten Druck von bis zu 20000 hPa, insbesondere von bis zu 10000 hPa, durchgeführt.

Bevorzugt beträgt die Dauer des erfindungsgemäßen ersten Schrittes weniger als 4 Stunden, besonders bevorzugt weniger als 2 Stunden, insbesondere 5 bis 60 Minuten.

Die im ersten Schritt des erfindungsgemäßen Verfahrens erhaltene Mischung hat eine Partikelgröße (Mittelwert der Volumenverteilung) von bevorzugt kleiner als 1 µm, besonders bevorzugt 100 bis 500 nm, insbesondere 100 bis 200 nm.

Im gegebenenfalls durchgeführten zweiten Schritt des erfindungsgemäßen Verfahrens wird die im ersten Schritt erhaltene Emulsion, insbesondere wenn sie hochviskos bis standfest ist, mit Wasser unter Rühren und/oder Homogenisieren verdünnt, so dass eine fließfähige Emulsion gebildet wird, die vorzugsweise mehr als 50 Teile Wasser je 100 Teile Komponente (a) enthält.

Das Rühren bzw. Homogenisieren kann unter den gleichen Bedingungen erfolgen wie für den ersten Schritt beschrieben.

Der zweite Schritt des erfindungsgemäßen Verfahrens wird bei Temperaturen von vorzugsweise 5 bis 50°C, insbesondere 10 bis 30°C, und dem Druck der umgebenden Atmosphäre, also zwischen 900 und 1100 hPa, oder bei einem erhöhten Druck von bis zu 20000 hPa, insbesondere von bis zu 10000 hPa, durchgeführt.

Der zweite Schritt kann in dem gleichen Behälter erfolgen wie der erste Verfahrensschritt.

Bevorzugt beträgt die Dauer des erfindungsgemäß gegebenenfalls durchgeführten zweiten Schrittes weniger als 4 Stunden, besonders bevorzugt weniger als 2 Stunden, insbesondere 5 bis 60 Minuten.

Beim erfindungsgemäßen Verfahren wird bevorzugt der zweite Schritt durchgeführt.

Im dritten Schritt des erfindungsgemäßen Verfahrens werden die Organopolysiloxane (a) kondensieren gelassen bis die Viskosität erreicht ist, wie sie für Siloxan (A) in der erfindungsgemäßen Emulsion gewünscht wird, also eine Viskosität von größer als 0,01m²/s (10 000 mm²/s), vorzugsweise größer als 0,1 m²/s (100 000 mm²/s), besonders bevorzugt größer als 1m²/s (1 000 000 mm²/s) jeweils bei 25°C.

Bevorzugt beträgt die Dauer des erfindungsgemäßen dritten Schritts 1 bis 200 Stunden, besonders bevorzugt 8 bis 96 Stunden, insbesondere 12 bis 72 Stunden. Der dritte Schritt kann in dem gleichen Behälter erfolgen wie der erste und zweite Schritt. Die Emulsion kann aber auch in einen speziellen Behälter überführt werden, wo gegebenenfalls mehrere hintereinander hergestellte Ansätze für den dritten Schritt vermischt werden. Es ist jedoch auch möglich den ersten und zweiten Schritt kontinuierlich und den dritten Schritt in einem Reifetank durchzuführen.

Der dritte Schritt des erfindungsgemäßen Verfahrens wird bei Temperaturen von vorzugsweise 2 bis 50°C, besonders bevorzugt 5 bis 30°C, insbesondere bei 5 bis 20°C, und einem Druck der umgebenden Atmosphäre, also zwischen 900 und 1100 hPa, durchgeführt.

Die bei dem erfindungsgemäßen Verfahren gegebenenfalls als Kondensationsnebenprodukte anfallenden Alkohole, z.B. wenn R³ in Formel (IV) verschieden Wasserstoffatom ist, können in der Emulsion verbleiben oder auch entfernt werden, beispielsweise durch Destillation unter Vakuum oder durch Extraktion.

Beispiele für die im gegebenenfalls durchgeführten vierten Schritt des erfindungsgemäßen Verfahrens eingesetzten Basen sind Alkalihydroxyde, wie NaOH und KOH, sowie Amine, wie z.B. Monoethanolamin und Triethanolamin. Der pH-Wert kann grundsätzlich auch durch die Zugabe von Alkalisalzen schwacher Säuren, wie z.B. Natriumcitrat, Natriumsilikat, Kaliumacetat oder Kaliumphosphat, eingestellt werden.

Bevorzugt handelt es sich bei den Basen, die im vierten Schritt des erfindungsgemäßen Verfahrens eingesetzt werden können, um Alkali- bzw. Erdalkalihydroxide, Ammoniak und Amine, besonders bevorzugt um NaOH, KOH, Monoethanolamin und Triethanolamin.

Der pH-Wert der Emulsion nach der erfindungsgemäßen Neutralisation beträgt vorzugsweise 5 bis 10, besonders bevorzugt 6 bis 8, insbesondere in etwa 7.

Der gegebenenfalls durchgeführte vierte Schritt des erfindungsgemäßen Verfahrens wird bei Temperaturen von vorzugsweise 5 bis 50°C, besonders bevorzugt 15 bis 30°C, und einem Druck der umgebenden Atmosphäre, also zwischen 900 und 1100 hPa, durchgeführt.

Beim erfindungsgemäßen Verfahren wird bevorzugt der vierte Schritt durchgeführt.

Die erfindungsgemäß erhaltenen Emulsionen können nun in einem gegebenenfalls durchgeführten 5. Schritt beliebig mit weiterem Wasser (d) und/oder weiteren Stoffen (e) vermischt werden.

Bevorzugt werden zusätzlich zu den Komponenten (a), (b), (c), (d) und gegebenenfalls (e) und Basen im erfindungsgemäßen Verfahren keine weiteren Komponenten eingesetzt.

Bei den im erfindungsgemäßen Verfahren eingesetzten Komponenten kann es sich jeweils um eine Art einer solchen Komponente wie auch um ein Gemisch aus mindestens zwei Arten einer jeweiligen Komponente handeln.

Die erfindungsgemäßen bzw. erfindungsgemäß hergestellten Emulsionen haben den Vorteil, dass sie hochviskose Polydiorganosiloxane enthalten und einen niedrigen Gehalt an Cyclen aufweisen.

Des Weiteren haben die erfindungsgemäßen bzw. erfindungsgemäß hergestellten Emulsionen den Vorteil, dass sie sehr stabil und damit lange haltbar sind.

Die erfindungsgemäßen bzw. erfindungsgemäß hergestellten Emulsionen haben den Vorteil, dass sie lagerstabil sind und ausgezeichnete anwendungstechnische Eigenschaften besitzen, wie z.B. eine sehr gute Wirkung als Trenn- und Gleitmittel, eine gute Benetzungsfähigkeit auf unterschiedlichen Substraten, eine gute Konditionierwirkung in Haarpflegeprodukten, d.h. deutliche Reduzierung der Nass- und Trockenkämmkraft.

Das erfindungsgemäße Verfahren hat den Vorteil, dass auf einfache und kostengünstige Art Emulsionen mit hochmolekularen Siloxanen hergestellt werden können.

Das erfindungsgemäße Verfahren hat ferner den Vorteil, dass auch nach längerer Dauer des dritten Schritts der Anteil an cyclischen Siloxanen gering bleibt, was z.B. bei einer kontinuierlichen Produktion mit einem breiteren Verweilzeitbereich besonders günstig ist.

Das erfindungsgemäße Verfahren hat den Vorteil, dass die Viskosität des Öles in einem weiten Bereich variiert und einfach eingestellt werden kann, ohne dass ein erhöhter Anteil an cyclischen Siloxanen gebildet wird.

Die erfindungsgemäßen bzw. erfindungsgemäß hergestellten Emulsionen sind für alle Zwecke einsetzbar, für die auch bisher Emulsionen mit hochviskosen Siloxanen eingesetzt worden sind, wie beispielsweise als Trennmittel, Gleitmittel, Hydrophobiermittel und zur Textilimprägnierung, bei der Verarbeitung von Gummi und Kunststoffen oder bei der Metallverarbeitung, Hydrophobiermittel für Glas und mineralische Baustoffe oder als Bestandteil von Körperpflegeprodukten.

Bei der Anwendung als Gleitmittel für Nähfäden können die erfindungsgemäßen Emulsionen z.B. mit Wachsemulsionen kombiniert werden. Mit den erfindungsgemäßen Emulsionen können Trennmittelformulierungen, wie z.B. für die Reifenindustrie, hergestellt werden, die neben der erfindungsgemäßen Emulsion weitere Komponenten wie Verdicker, wie Xanthan Gum oder Polyacrylate, Füllstoffe, wie Talkum oder Glimmer, Wachse und weitere dem Fachmann bekannte Komponenten enthalten. Die hohe Viskosität und der niedrige Gehalt an flüchtigen Siloxanen ist bei diesen Anwendungen besonders vorteilhaft.

Ein weiterer Gegenstand der Erfindung sind Körperpflegemittel enthaltend erfindungsgemäße Emulsionen in Mengen von 0,05 bis 10 Gew.-%, besonders bevorzugt 0,5 bis 5 Gew.-%.

Vorzugsweise handelt es sich bei den erfindungsgemäßen Körperpflegemitteln um Haarpflegemittel.

Diese Haarpflegemittel enthalten neben den erfindungsgemäßen bzw. erfindungsgemäß hergestellten Emulsionen bevorzugt einen oder mehrere Konditionierer ausgewählt z.B. aus der Gruppe quartärnärer Ammonimverbindungen, natürlicher oder synthetischer Wachse, Pflanzenölen, Mineralölen, fluorierten Ölen, Siliconölen, insbesondere Aminosiliconölen, organischen Polymeren und Copolymeren, die nichtionisch, anionisch, kationisch oder amphoter sein können, kationischen Proteine und kationischen Tensiden.

Weitere Inhaltsstoffe dieser Haarpflegemittel sind z.B. Wasser, Tenside, Fettalkohole, Rheologiemodifizierer, Pearlizer, organische Säuren, Duftstoffe, Konservierer, Vitamine, Sonnenschutzmittel, Salze, Farbstoffe sowie weitere dem Fachmann bekannte Komponenten von Haarpflegemitteln.

Bei den die erfindungsgemäßen bzw. erfindungsgemäß hergestellten Emulsionen enthaltenden Haarpflegemitteln kann es sich z.B. um Shampoos, Haarmasken, Haarspülungen, Haarwachse, Haarcremes, Haargele, Haarschäume, Haarsprays und Haarfärbemittel handeln. Diese Pflegemitteln verbessern sowohl die Trocken- und die Nasskämmbarkeit als auch das Griffgefühl im nassen und trockenen Haar.

Die Anwendung kann z.B. beim Waschen, nach dem Waschen, als Vor- oder Nachbehandlung beim Bleichen oder beim Färben mit Direkt- oder Oxidationsfarbstoffen und bei der permanenten Verformung der Haare (z.B. Dauerwelle) erfolgen.

Ein weiterer Gegenstand der Erfindung sind Haarpflegemittel enthaltend erfindungsgemäße Emulsionen sowie mindestens einen Konditionierer.

In den nachfolgenden Beispielen beziehen sich alle Angaben von Teilen und Prozentsätzen, soweit nicht anders angegeben, auf das Gewicht. Sofern nicht anders angegeben, werden die folgenden Beispiele bei einem Druck der umgebenden Atmosphäre, also bei etwa 1010 hPa, und bei Raumtemperatur, also etwa 25°C bzw. einer Temperatur, die sich beim Zusammengeben der Reaktanten bei Raumtemperatur ohne zusätzliche Heizung oder Kühlung einstellt, durchgeführt. Alle in den Beispielen angeführten Viskositätsangaben beziehen sich auf eine Temperatur von 25°C.

Die in den nachfolgenden Beispielen hergestellten Emulsionen wurden wie folgt geprüft:

Die Teilchengröße wurde an mit einem Partikelgrößenanalysator Zetasizer ZEN1600/ Nano-S, Fa. Malvern, Software Version 6.01 mittels dynamischer Lichtstreuung bestimmt. Dazu wurden die Emulsionen mit gefilterten und entgasten Wasser auf 0,5% verdünnt. Die angegebenen Werte beziehen sich immer auf den Wert D(50).

Zur Bestimmung der Ölviskosität wurden 20 g Emulsion mit 30 g Aceton versetzt, wobei sich die Emulsion trennte. Die Aceton/ Wasserphase wurde abgetrennt und der Vorgang noch einmal wiederholt. Anschließend wurde das Polymer drei Mal mit Wasser gewaschen und bei 110°C unter Rühren getrocknet, bis keine Wassertröpfchen mehr zu sehen waren und anschließend noch 8h bei 110°C im Trockenschrank nachbehandelt. Die Viskosität wurde mit einem Kegel-Platte-Viskosimeter MCR 300 (Paar-Physika) bei 25°C und einem Schergefälle von 1/s bestimmt.

Zur Bestimmung des Gehaltes an Octamethylcyclotetrasiloxan (D₄) wurde ein ²⁹Si-NMR Spektrum der Emulsion aufgenommen (Avance 400 Fa. Bruker, 10 mm selektiver ²⁹Si NMR-Probenkopf, Zusatz von 15% D₂O zur Originalemulsion, Pulswinkel 30°, Wartezeit 30 s, 400 scans).

Aus den Integralen der Signale zwischen -19,75 bis -20 ppm (D₄) und -21,5 bis -23,25 (restliche D-Einheiten) wurde der D₄-Anteil in mol% Si ermittelt, der aufgrund der gleichen Molmasse der einzelnen Siloxaneinheit (74g/mol) praktisch gleich dem Anteil an D₄ in Gew.-% bezogen auf Polydimethylsiloxane ist.

### Beispiel 1

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 10 Teile eines 2-Ethylhexylphosphats mit einer Säurezahl von 295 mg KOH/g (erhältlich unter dem Namen "Servoxyl VPTZ 100" bei Elementis Specialties Netherlands B.V. Delden), 14 Teile eines ethoxylierten Stearylalkohols der Formel C₁₈H₃₇-O-(CH₂CH₂O)₂₀-H erhältlich unter dem Namen "Arlypon SA 20" bei der Cognis AG, Düsseldorf) und 10 Teile Wasser zugegeben und 10 min homogenisiert. Die entstehende gelartige Phase (Fließgrenze 920 Pa) mit einer Teilchengröße von kleiner als 250 nm wird innerhalb von 10 min mit 100 Teilen Wasser verdünnt und bei 10°C gelagert. Diese Emulsion hatte einen pH-Wert von 2,6. Nach 24 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 2

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 10 Teile eines Isononylphosphats mit einer Säurezahl von 300 mg KOH/g (erhältlich unter dem Namen "Servoxyl VPXZ 100" bei Elementis Specialties Netherlands B.V. Delden), 10 Teile eines ethoxylierten Laurylalkohols der Formel C₁₂H₂₃-O-(CH₂CH₂O)₂₃-H (erhältlich unter dem Namen "Brij 35" bei der Croda GmbH, D-Nettetal) und 10 Teile Wasser 5 min homogenisiert. Die entstehende gelartige Phase mit einer Fließgrenze von 1730 Pa und einer Partikelgröße von kleiner als 200 nm wird innerhalb von 10 min mit 100 Teilen Wasser verdünnt und bei 15°C gelagert. Diese Emulsion hatte einen pH-Wert von 2,3. Nach 24 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 3

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 10 Teile eines 2-Ethylhexylphosphats mit einer Säurezahl von 295 mg KOH/g (erhältlich unter dem Namen "Servoxyl VPTZ 100" bei Elementis Specialties Netherlands B.V. Delden), 10 Teile eines ethoxylierten Laurylalkohols der Formel C₁₂H₂₃-O- (CH₂CH₂O)₂₃-H (erhältlich unter dem Namen "Brij 35" bei der Croda GmbH, D-Nettetal) und 10 Teile Wasser zugegeben und 10 min homogenisiert. Die entstehende gelartige Phase (Fließgrenze 1340 Pa) mit einer Partikelgröße von kleiner als 200 nm wird innerhalb von 10 min mit 100 Teilen Wasser verdünnt und bei 10°C gelagert. Diese Emulsion hatte einen pH-Wert von 2,2. Nach 24 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 4

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 6 Teile eines 2-Ethylhexylphosphats mit einer Säurezahl von 225 mg KOH/g (erhältlich unter dem Namen "Servoxyl VPDZ 100" bei Elementis Specialties Netherlands B.V. Delden), 10 Teile eines ethoxylierten Laurylalkohols der Formel C₁₂H₂₃-O-(CH₂CH₂O)₂₃-H (erhältlich unter dem Namen "Brij 35" bei der Croda GmbH, D-Nettetal) und 10 Teile Wasser zugegeben und 15 min homogenisiert. Die entstehende gelartige Phase (Fließgrenze 990 Pa) mit einer Partikelgröße von kleiner als 200 nm wird innerhalb von 10 min mit 100 Teilen Wasser verdünnt und bei 10°C gelagert. Diese Emulsion hatte einen pH-Wert von 2,5. Nach 24 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 5

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 4 Teile eines n-Butylphosphats mit einer Säurezahl von 465 mg KOH/g (erhältlich unter dem Namen "Servoxyl VPIZ 100" bei Elementis Specialties Netherlands B.V. Delden), 10 Teile eine ethoxylierten Stearinsäure der Formel C₁₆H₃₃CH₂C(O)-O-(CH₂CH₂O)₄₀-H (erhältlich unter dem Namen "Myrj 52S" bei der Croda GmbH, D-Nettetal) und 10 Teile Wasser zugegeben und 15 min homogenisiert. Die entstehende gelartige Phase (Fließgrenze 720 Pa) mit einer Partikelgröße von kleiner als 200 nm wird innerhalb von 10 min mit 100 Teilen Wasser verdünnt und bei 10°C gelagert. Diese Emulsion hatte einen pH-Wert von 1,8. Nach 96 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 6

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 10 Teile eines Octyl-Decylphosphats mit einer Säurezahl von 295 mg KOH/g (erhältlich unter dem Namen "Crodafos 810 A" bei der Croda GmbH, D-Nettetal), 14 Teile eines ethoxylierten Laurylalkohols der Formel C₁₂H₂₃-O- (CH₂CH₂O)₂₃-H (erhältlich unter dem Namen "Brij 35" bei der Croda GmbH, D-Nettetal) und 10 Teile Wasser zugegeben und 10 min homogenisiert. Die entstehende gelartige Phase (Fließgrenze 1940 Pa) mit einer Partikelgröße von kleiner als 200 nm wird innerhalb von 10 min mit 100 Teilen Wasser verdünnt und bei 10°C gelagert. Diese Emulsion hatte einen pH-Wert von 2,0. Nach 24 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 7

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 6 Teile eines Octyl-Decylphosphats mit einer Säurezahl von 295 mg KOH/g (erhältlich unter dem Namen "Crodafos 810 A" bei der Croda GmbH, D-Nettetal), 10 Teile eines ethoxylierten Laurylalkohols der Formel C₁₂H₂₃-O- (CH₂CH₂O)₂₃-H (erhältlich unter dem Namen "Brij 35" bei der Croda GmbH, D-Nettetal) und 10 Teile Wasser zugegeben und 10 min homogenisiert. Die entstehende gelartige Phase (Fließgrenze 1110 Pa) mit einer Partikelgröße von kleiner als 200 nm wird innerhalb von 10 min mit 100 Teilen Wasser verdünnt und bei 15°C gelagert. Diese Emulsion hatte einen pH-Wert von 2,1. Nach 24 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 8

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 6 Teile eines Octyl-Decylphosphats mit einer Säurezahl von 295 mg KOH/g (erhältlich unter dem Namen "Crodafos 810 A" bei der Croda GmbH, D-Nettetal), 10 Teile eines ethoxylierten Sorbitanlaurates (erhältlich unter dem Namen "Tween 20" bei der Croda GmbH, D-Nettetal) und 10 Teile Wasser zugegeben und 10 min homogenisiert. Die entstehende gelartige Phase (Fließgrenze 820 Pa) mit einer Partikelgröße von kleiner als 300 nm wird innerhalb von 10 min mit 100 Teilen Wasser verdünnt und bei 15°C gelagert. Diese Emulsion hatte einen pH-Wert von 2,0. Nach 24 h wird die Emulsion mit Triethanolamin auf einen pH-Wert von 7 eingestellt und 0,18 Teile Konservierungsmittel auf der Basis von Isothiazolinonen (erhältlich unter der Bezeichnung "Kathon CG" bei Acima Chemical Industries Ltd., CH-9471 Buchs/SG) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beisplel 9

100 Teile eines mit α,ω-hydroxyterminierten Polydimethylsiloxans mit einer Viskosität von 60 mPas werden in einem Becher vorgelegt. Mit einem Rotor-Stator-Homogenisator (Ultra-Turrax, Umfangsgeschwindigkeit 16 m/s) werden 6 Teile eines Octyl-Decylphosphats mit einer Säurezahl von 295 mg KOH/g (erhältlich unter dem Namen "Crodafos 810 A" bei der Croda GmbH, D-Nettetal), 10 Teile eines ethoxylierten Rizinusöls (erhältlich unter dem Namen "Atlas G1300" bei der Croda GmbH, D-Nettetal) und 10 Teile Wasser zugegeben und 10 min homogenisiert. Die entstehende gelartige Phase (Fließgrenze 900 Pa) mit einer Partikelgröße von kleiner als 200 nm wird innerhalb von 10 min mit 100 Teilen Wasser verdünnt und bei 15°C gelagert. Diese Emulsion hatte einen pH-Wert von 2,1. Nach 24 h wird die Emulsion mit Triethanolamin auf einen pH-Wert von 7 eingestellt und 0,24 Teile Konservierungsmittel auf der Basis von Methylisothiazolinonen und Ethylhexylglycerin (erhältlich unter der Bezeichnung "Euxyl K220" bei Schülke & Mayr GmbH, Norderstedt) zugesetzt.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

### Beispiel 10

950 kg eines mit α,ω-hydroxyterminierten Polydimethylsiloxans mit einer Viskosität von 60 mPas werden in einem Mischrührwerk mit einem Volumen von 2000 l (Becomix RW 2000) vorgelegt. Der Homogenisator wird angeschaltet und auf eine Umfangsgeschwindigkeit von 24 m/s eingestellt. 60 kg eines Octyl-Decylphosphats mit einer Säurezahl von 295 mg KOH/g (erhältlich unter dem Namen "Crodafos 810 A" bei der Croda GmbH, D-Nettetal), 100 kg eines ethoxylierten Laurylalkohols der Formel C₁₂H₂₃-O-(CH₂CH₂O)₂₃-H (erhältlich unter dem Namen "Brij 35" bei der Croda GmbH, D-Nettetal) und 100 kg Wasser werden zugegeben und 15 min homogenisiert. Es wurde eine standfeste gelartige Phase gebildet, die eine Fließgrenze von 1050 Pa hatte. Diese Phase wurde weitere 45 min homogenisiert, bis eine Teilchengröße kleiner 500 nm erreicht wurde. Anschließend wurde die Emulsion innerhalb von 10 min mit 900 kg Wasser verdünnt und bei 15°C gelagert. Diese Emulsion hatte einen pH-Wert von 1,3. Nach 48 h wird die Emulsion mit Triethanolamin auf einen pH-Wert von 7 eingestellt. Anschließend wurden 1,8 kg Konservierungsmittel auf der Basis von Isothiazolinonen (erhältlich unter der Bezeichnung "Kathon CG" bei Acima Chemical Industries Ltd. CH-9471 Buchs/SG) zugegeben.

Die so erhaltene Emulsion wird nun hinsichtlich Teilchengröße, Ölviskosität und den Gehalt an Octamethylcyclotetrasiloxan D₄ untersucht. Die Ergebnisse finden sich in Tabelle 1.

**Tabelle 1:**

| Beispiel | Teilchengröße D(50) in nm | Ölviskosität in Pas | D₄ in Gew.-% |
|---|---|---|---|
| 1 | 205 | 1790 | < 0,05 |
| 2 | 153 | 762 | 0,05 |
| 3 | 143 | 1430 | 0,1 |
| 4 | 175 | 1050 | 0,05 |
| 5 | 162 | 1630 | 0,15 |
| 6 | 116 | 1220 | 0,05 |
| 7 | 154 | 2630 | 0,05 |
| 8 | 214 | 1730 | 0,1 |
| 9 | 174 | 1420 | 0,05 |
| 10 | 156 | 1560 | 0,08 |

Alle erfindungsgemäßen Emulsionen waren sehr stabil, sie zeigten weder beim Zentrifugieren (1h bei 2500g) noch bei der Lagerung über 6 Monate Abscheidungen. Die Partikelgröße war auch nach 28d 50°C unverändert.

### Beispiel 11

Ein Shampoo 19:3 wird wie folgt formuliert, wobei die einzelnen Komponenten entsprechend der INCI-Nomenklatur bezeichnet werden:
0,2 Teile Guar Hydroxypropyltrimoniumchlorid (erhältlich unter dem Namen N-Hance^{®} 3000 bei der Hercules Inc.) werden in 11,98 Teilen Wasser dispergiert. 71,7 Teile Sodium Laureth Sulfat (erhältlich unter dem Namen Genapol^{®} LRO 26,5% bei der Clariant GmbH) werden langsam eingerührt und die Mischung wird auf 75°C erwärmt. Dabei werden 0,3 Teile PEG-150 Distearate (erhältlich unter dem Namen Emulgin^{®} EO 33 bei der Cognis Deutschland GmbH) bei Erreichen von 50°C zugegeben und wenn 65°C erreicht sind 1,2 Teile Glycol Distearate (erhältlich unter dem Namen Genapol^{®} PMS bei der Clariant GmbH). Die Mischung wird gemischt bis 75°C erreicht sind. Dann wird die Mischung abgekühlt. Wenn 35°C erreicht sind, werden 0,06 Teile Konservierer Methylchloroisothiazolinon/Methylisothiazolinon (erhältlich unter dem Namen Kathon™ CG bei der Rohm&Haas Company, Inc.) und 4 Teile der Emulsion von Beispiel 6 zugegeben und 5 Minuten gerührt. Abschließend werden 10,06 Teile Cocamidopropyl Betaine (erhältlich unter dem Namen Genagen^{®} CAB 30% bei der Clariant GmbH) und 0,5 Teile Natriumchlorid 25% zugegeben und jeweils 10 Minuten gerührt.
   Das so erhaltene Shampoo verbessert sowohl die Trocken- und die Nasskämmbarkeit als auch das Griffgefühl im nassen und trockenen Haar.

### Beispiel 12

Ein Shampoo 11:4 wird wie folgt formuliert, wobei die einzelnen Komponenten entsprechend der INCI-Nomenklatur bezeichnet werden:
0,1 Teile Polyquaternium-10 (erhältlich unter dem Namen Ucare™ Polymer JR-400 bei der Amerchol Corporation) werden in 39,04 Teilen Wasser dispergiert. 41,5 Teile Sodium Laureth Sulfat (erhältlich unter dem Namen Genapol^{®} LRO 26,5% bei der Clariant GmbH) werden langsam eingerührt und die Mischung wird auf 75°C erwärmt. Dabei werden 0,2 Teile Hydroxyethylcellulose (erhältlich unter dem Namen Tylose^{®} H 4000 P2 bei der Shin-Etsu Chemical Co.) bei Erreichen von 50°C zugegeben und wenn 65°C erreicht sind 1,2 Teile Glycol Distearate (erhältlich unter dem Namen Genapol^{®} PMS bei der Clariant GmbH). Die Mischung wird gemischt bis 75°C erreicht sind. Dann wird die Mischung abgekühlt. Wenn 35°C erreicht sind, werden 0,06 Teile Konservierer Methylchloroisothiazolinon/Methylisothiazolinon (erhältlich unter dem Namen Kathon™ CG bei der Rohm&Haas Company, Inc.) und 4 Teile der Emulsion von Beispiel 6 zugegeben und 5 Minuten gerührt. Abschließend werden 13,4 Teile Cocamidopropyl Betaine (erhältlich unter dem Namen Genagen^{®} CAB 30% bei der Clariant GmbH) und 0,5 Teile Natriumchlorid 25% zugegeben und jeweils 10 Minuten gerührt.
   Das so erhaltene Shampoo verbessert sowohl die Trocken- und die Nasskämmbarkeit als auch das Griffgefühl im nassen und trockenen Haar.

### Beispiel 13

Ein Conditioner wird wie folgt formuliert, wobei die einzelnen Komponenten entsprechend der INCI-Nomenklatur bezeichnet werden:
87,04 Teile Wasser werden vorgelegt und unter Rühren auf 75°C erwärmt. Dabei werden 1,2 Teile Hydroxyethylcellulose (erhältlich unter dem Namen Tylose^{®} H 4000 P2 bei der Shin-Etsu Chemical Co.) zugegeben. Wenn 65°C erreicht sind, werden 0,5 Teile Stearamidopropyl Dimethylamin (erhältlich unter dem Namen Incromine™ SB bei der Croda GmbH), 1 Teil Polysorbate 80 (erhältlich unter dem Namen Tween™ 80 bei der Croda GmbH), 3 Teile Stearyl Alcohol (erhältlich unter dem Namen Stearylalkohol bei der Merck-Schuchardt), 1 Teil Cetyl Alcohol (erhältlich unter dem Namen Cetylalkohol bei der Merck KGaA) und 1,76 Teile Behentrimonium Chlorid (erhältlich unter dem Namen Genamin^{®} KDMP bei der Clariant GmbH) zugegeben. Die Mischung wird gemischt bis 75°C erreicht sind. Dann wird die Mischung abgekühlt. Während des Abkühlens werden 0,2 Teile Citric Acid (erhältlich unter dem Namen Citric Acid bei Sigma) und 0,2 Teile Tetrasodium EDTA (erhältlich unter dem Namen EDETA^{®} B Pulver bei der BASF Corporation) zugegeben. Wenn 35°C erreicht sind, werden 0,1 Teile Konservierer Methylchloroisothiazolinon/Methylisothiazolinon (erhältlich unter dem Namen Kathon™ CG bei der Rohm&Haas Company, Inc.) und 4 Teile der Emulsion von Beispiel 6 zugegeben und 5 Minuten gerührt. Abschließend wird eine Minute unter dem Turrax homogenisiert.
Der so erhaltene Conditioner verbessert sowohl die Trocken- und die Nasskämmbarkeit als auch das Griffgefühl im nassen und trockenen Haar.

## Patentansprüche

1. Emulsionen von Polyorganosiloxanen enthaltend
(A) Polyorganosiloxane, die eine Viskosität größer als 0,01 m²/s (10 000 mm²/s), gemessen bei 25°C, aufweisen,
(B) mindestens einen Emulgator der Formel
(RO)ₙP(O)(OH)₍₃₋ₙ₎ (I),
worin
R gleich oder verschieden sein kann und einwertige Kohlenwasserstoffreste mit 4 bis 30 Kohlenstoffatomen bedeutet n 1 oder 2 ist,
und/oder dessen Salze,
(C) mindestens einen Emulgator ausgewählt aus der Gruppe bestehend aus
(C1) ethoxylierten Triglyceriden mit 40 bis 400 Ethylenglycolgrupppen,
(C2) ethoxylierten sorbitanestern von Fettsäuren mit 12 bis 18 Kohlenstoffatomen und 10 bis 40 Ethylenglycolgruppen,
(C3) Verbindungen der Formel
R¹-O-(CH₂CH₂O)ₘ-H (II)
und
(C4) Verbindungen der Formel
R³CH₂C(O)-O-(CH₂CH₂O)ₚ-H (III),
worin
R¹ einen linearen Alkylrest mit 10 bis 30 Kohlenstoffatomen bedeutet,
R² einen Alkylrest mit 10 bis 30 Kohlenstoffatomen bedeutet,
m einen Wert von 15 bis 100 darstellt und
p einen Wert von 15 bis 100 annimmt,
sowie
(D) Wasser,
mit der Maßgabe, dass die Emulsionen weniger als 2 Ges.-% Octaorganylcyclotetrasiloxan (D₄), bezogen auf Komponente (A),
enthalten.

2. Emulsionen gemäß Anspruch 1, **dadurch gekennzeichnet, dass** sie weniger als 1 Gew.-% Octaorganylcyclotetrasiloxan (D₄), bezogen auf Komponente (A), enthalten.

3. Emulsionen gemäß Anspruch 1 oder 2, **dadurch gekennzeichnet, dass** Polyorganosiloxane (A) eine Viskosität größer als 100 000 mm²/s, gemessen bei 25°C, aufweisen.

4. Emulsionen gemäß einem oder mehreren der Ansprüche 1 bis 3, **dadurch gekennzeichnet, dass** sie einen Teilchendurchmesser von 50 bis 1000 nm aufweisen.

5. Emulsionen gemäß einem oder mehreren der Ansprüche 1 bis 4, **dadurch gekennzeichnet, dass** es sich bei Komponente (C) um Verbindungen der Formel (II) handelt.

6. Verfahren zur Herstellung der Emulsionen gemäß einem oder mehreren der Ansprüche 1 bis 5, **dadurch gekennzeichnet, dass**
(a) Polyorganosiloxane enthaltend Einheiten der allgemeinen Formel
R⁴ₐ(R³O)_{b}SiO_{(4-a-b)/2} (IV),
worin
R⁴ gleich oder verschieden sein kann und einen einwertigen, gegebenenfalls substituierten Kohlenwasserstoffrest mit 1 bis 30 Kohlenstoffatomen oder Wasserstoffatom bedeutet,
R³ gleich oder verschieden sein kann und Wasserstoffatom oder einen einwertigen, gegebenenfalls substituierten Kohlenwasserstoffrest bedeutet,
a 0, 1, 2 oder 3 ist und
b 0, 1, 2 oder 3 ist,
mit der Maßgabe, dass die Summe a+b kleiner oder gleich 3 ist und die Polyorganosiloxane 5 bis 500 Einheiten der Formel (IV) enthalten, wobei in mindestens einer Einheit b verschieden 0 ist,
(b) Emulgator der Formel (I), dessen OH-Gruppen gegebenenfalls teilweise neutralisiert sein können,
(c) Emulgator ausgewählt aus der Gruppe bestehend aus ethoxylierten Triglyceriden mit 40 bis 400 Ethylenglycolgruppen, ethoxylierten Sorbitanester von Fettsäuren mit 12 bis 18 Kohlenstoffatomen und 10 bis 40 Ethylenglycolgruppen, Verbindungen der Formel (II) und Verbindungen der Formel (III),
(d) Wasser und
gegebenenfalls
(e) weitere Stoffe
durch Rühren und/oder Homogenisieren vermischt werden sowie die Organopolysiloxane (a) enthaltend Einheiten der Formel (IV) bei Temperaturen von 0 bis 50°C kondensieren gelassen werden bis die gewünschte Viskosität erreicht ist und gegebenenfalls anschließend der Emulgator der Formel (I) mit Basen neutralisiert wird, so dass der pH-Wert der Emulsion größer als 5 ist, und gegebenenfalls weiteres Wasser (d) und/oder weitere Stoffe (e) zugegeben werden.

7. verfahren nach Anspruch 6, **dadurch gekennzeichnet, dass** Komponente (c) in Mengen von 1 bis 25 Gewichtsteilen, bezogen auf 100 Gewichtsteile Polyorganosiloxan (a), eingesetzt wird.

8. Verfahren nach Anspruch 6 oder 7, **dadurch gekennzeichnet, dass**
in einem 1. Schritt
(a) 100 Gewichtsteile Polyorganosiloxane enthaltend Einheiten der allgemeinen Formel
R⁴ₐ(R²O)_{b}SiO_{(4-a-b)/2} (IV),
worin
R⁴ gleich oder verschieden sein kann und einen einwertigen, gegebenenfalls substituierten Kohlenwasserstoffrest mit 1 bis 30 Kohlenstoffatomen oder Wasserstoffatom bedeutet,
R³ gleich oder verschieden sein kann und Wasserstoffatom oder einen einwertigen, gegebenenfalls substituierten Kohlenwasserstoffrest bedeutet,
a 0, 1, 2 oder 3 ist und
b 0, 1, 2 oder 3 ist,
mit der Maßgabe, dass die summe a+b kleiner oder gleich 3 ist und die Organopolysiloxane 5 bis 500 Einheiten der Formel (IV) enthalten, wobei in mindestens einer Einheit b verschieden 0 ist,
(b) 1 bis 30 Gewichtsteile eines Emulgators der Formel (I), dessen OH-Gruppen gegebenenfalls teilweise neutralisiert sein können,
(c) 1 bis 30 Gewichtsteile eines Emulgators ausgewählt aus der Gruppe bestehend aus ethoxylierten Triglyceriden mit 40 bis 400 Ethylenglycolgruppen, ethoxylierten Sorbitanestern von Fettsäuren mit 12 bis 18 Kohlenstoffatomen und 10 bis 40 Ethylenglycolgruppe, Verbindungen der Formel (II) und Verbindungen der Formel (III),
(d) 1 bis 50 Gewichststeile Wasser und
gegebenenfalls
(e) weitere Stoffe
durch Rühren und/oder Homogenisieren vermischt werden,
in einem gegebenenfalls durchgeführten 2. Schritt weiteres Wasser (d) zugegeben wird,
in einem 3. Schritt
die Organopolysiloxanne (a) enthaltend Einheiten der Formel (IV) bei Temperaturen von 0 bis 50°C kondensieren gelassen werden bis die gewünschte Viskosität erreicht ist,
in einem gegebenenfalls durchgeführten 4. Schritt der Emulgator der Formel (I) mit Basen neutralisiert wird, so dass der pH-Wert der Emulsion größer als 5 ist und
in einem gegebenenfalls durchgeführten 5. Schritt die im 4. Schritt erhaltene Emulsion mit weiterem Wasser (d) und/oder weiteren Stoffen (e) vermischt wird.

9. Körperpflegemittel enthaltend Emulsionen gemäß einem oder mehreren der Ansprüche 1 bis 5 und/oder hergestellt nach einem oder mehreren der. Ansprüche 6 bis B in Mengen von 0,05 bis 10 Gew.-%.

10. Körperpflegemittel gemäß Anspruch 9, **dadurch gekennzeichnet, dass** es sich um ein Haarpflegemittel handelt und mindestens einen Konditionierer enthält.

## Claims

1. Emulsions of polyorganosiloxanes comprising
(A) polyorganosiloxanes having a viscosity greater than 0.01 m²/s (10 000 mm²/s), measured at 25°C,
(B) at least one emulsifier of the formula
(RO)ₙP(O)(OH)₍₃₋ₙ₎ (I),
in which
R may be the same or different and denotes monovalent hydrocarbyl radicals having 4 to 30 carbon atoms,
n is 1 or 2,
and/or salts thereof,
(C) at least one emulsifier selected from the group consisting of
(C1) ethoxylated triglycerides having 40 to 400 ethylene glycol groups,
(C2) ethoxylated sorbitan esters of fatty acids having 12 to 18 carbon atoms and 10 to 40 ethylene glycol groups,
(C3) compounds of the formula
R¹-O-(CH₂CH₂O)ₘ-H (II)
and
(C4) compounds of the formula
R²CH₂C(O)-O-(CH₂CH₂O)ₚ-H (III),
in which
R¹ is a linear alkyl radical having 10 to 30 carbon atoms,
R² is an alkyl radical having 10 to 30 carbon atoms,
m is a value from 15 to 100 and
p assumes a value from 15 to 100,
and
(D) water,
with the proviso that the emulsions contain less than 2% by weight of octaorganylcyclotetrasiloxane (D₄), based on component (A).

2. Emulsions according to Claim 1, **characterized in that** they contain less than 1% by weight of octaorganylcyclotetrasiloxane (D₄), based on component (A).

3. Emulsions according to Claim 1 or 2, **characterized in that** polyorganosiloxanes (A) have a viscosity greater than 100 000 mm²/s, measured at 25°C.

4. Emulsions according to one or more of Claims 1 to 3, **characterized in that** they have a particle diameter of 50 to 1000 nm.

5. Emulsions according to one or more of Claims 1 to 4, **characterized in that** component (C) comprises compounds of the formula (II).

6. Process for producing the emulsions according to one or more of Claims 1 to 5, **characterized in that**
(a) polyorganosiloxanes containing units of the general formula
R⁴ₐ(R³O)_{b}SiO_{(4-a-b)/2} (IV),
in which
R⁴ may be the same or different and is a monovalent, optionally substituted hydrocarbyl radical having 1 to 30 carbon atoms or hydrogen atom,
R³ may be the same or different and is a hydrogen atom or a monovalent, optionally substituted hydrocarbyl radical,
a is 0, 1, 2 or 3 and
b is 0, 1, 2 or 3,
with the proviso that the sum of a+b is less than or equal to 3,
and the polyorganosiloxanes contain 5 to 500 units of the formula (IV), where b is not 0 in at least one unit,
(b) emulsifier of the formula (I), wherein the OH groups may optionally be partly neutralized,
(c) emulsifier selected from the group consisting of ethoxylated triglycerides having 40 to 400 ethylene glycol groups, ethoxylated sorbitan esters of fatty acids having 12 to 18 carbon atoms and 10 to 40 ethylene glycol groups, compounds of the formula (II) and compounds of the formula (III),
(d) water and
optionally
(e) further substances
are mixed by stirring and/or homogenizing, and the organopolysiloxanes (a) containing units of the formula (IV) are allowed to condense at temperatures of 0 to 50°C until the desired viscosity has been attained and then the emulsifier of the formula (I) is optionally neutralized with bases, such that the pH of the emulsion is greater than 5, and optionally further water (d) and/or further substances (e) are added.

7. Process according to Claim 6, **characterized in that** component (c) is used in amounts of 1 to 25 parts by weight, based on 100 parts by weight of polyorganosiloxane (a).

8. Process according to Claim 6 or 7, **characterized in that**
in a 1st step
(a) 100 parts by weight of polyorganosiloxanes containing units of the general formula
R⁴ₐ(R³O)_{b}SiO_{(4-a-b)/2} (IV),
in which
R⁴ may be the same or different and is a monovalent, optionally substituted hydrocarbyl radical having 1 to 30 carbon atoms or hydrogen atom,
R³ may be the same or different and is a hydrogen atom or a monovalent, optionally substituted hydrocarbyl radical,
a is 0, 1, 2 or 3 and
b is 0, 1, 2 or 3,
with the proviso that the sum of a+b is less than or equal to 3,
and the organopolysiloxanes contain 5 to 500 units of the formula (IV), where b is not 0 in at least one unit,
(b) 1 to 30 parts by weight of an emulsifier of the formula (I), wherein the OH groups may optionally be partly neutralized,
(c) 1 to 30 parts by weight of an emulsifier selected from the group consisting of ethoxylated triglycerides having 40 to 400 ethylene glycol groups, ethoxylated sorbitan esters of fatty acids having 12 to 18 carbon atoms and 10 to 40 ethylene glycol groups, compounds of the formula (II) and compounds of the formula (III),
(d) 1 to 50 parts by weight of water and
optionally
(e) further substances
are mixed by stirring and/or homogenizing,
in an optional 2nd step
further water (d) is added,
in a 3rd step
the organopolysiloxanes (a) containing units of the formula (IV) are allowed to condense at temperatures of 0 to 50°C until the desired viscosity has been attained,
in an optional 4th step
the emulsifier of the formula (I) is neutralized with bases, such that the pH of the emulsion is greater than 5, and
in an optional 5th step
the emulsion obtained in the 4th step is mixed with further water (d) and/or further substances (e).

9. Personal care composition comprising emulsions according to one or more of Claims 1 to 5 and/or produced according to one or more of Claims 6 to 8 in amounts of 0.05 to 10% by weight.

10. Personal care composition according to Claim 9, **characterized in that** it is a haircare composition and comprises at least one conditioner.

## Revendications

1. Émulsions de polyorganosiloxanes contenant
(A) des polyorganosiloxanes qui présentent une viscosité supérieure à 0,01 m²/s (10 000 mm²/s), mesurée à 25 °C,
(B) au moins un émulsifiant de formule
(RO)ₙP(O)(OH)₍₃₋ₙ₎ (I)
dans laquelle
les R peuvent être identiques ou différents, et signifient des radicaux hydrocarbonés monovalents de 4 à 30 atomes de carbone,
n représente 1 ou 2,
et/ou ses sels,
(C) au moins un émulsifiant choisi dans le groupe constitué par
(C1) les triglycérides éthoxylés contenant 40 à 400 groupes éthylène glycol,
(C2) les esters de sorbitane d'acides gras éthoxylés contenant 12 à 18 atomes de carbone et 10 à 40 groupes éthylène glycol,
(C3) les composés de formule
R¹-O-(CH₂CH₂O)ₘ-H (II)
et
(C4) les composés de formule
R²CH₂C(O)-O-(CH₂CH₂O)ₚ-H (III)
dans lesquelles
R¹ signifie un radical alkyle linéaire de 10 à 30 atomes de carbone,
R² signifie un radical alkyle de 10 à 30 atomes de carbone,
m représente une valeur de 15 à 100 et
p prend une valeur de 15 à 100,
ainsi que
(D) de l'eau,
à condition que les émulsions contiennent moins de 2 % en poids d'octaorganylcyclotétrasiloxane (D₄), par rapport au composant (A).

2. Émulsions selon la revendication 1, **caractérisées en ce qu'**elles contiennent moins de 1 % en poids d'octaorganylcyclotétrasiloxane (D₄), par rapport au composant (A).

3. Émulsions selon la revendication 1 ou 2, **caractérisées en ce que** les polyorganosiloxanes (A) présentent une viscosité supérieure à 100 000 mm²/s, mesurée à 25 °C.

4. Émulsions selon une ou plusieurs des revendications 1 à 3, **caractérisées en ce qu'**elles présentent un diamètre de particule de 50 à 1 000 nm.

5. Émulsions selon une ou plusieurs des revendications 1 à 4, **caractérisées en ce que** le composant (C) consiste en des composés de formule (II).

6. Procédé de fabrication des émulsions selon une ou plusieurs des revendications 1 à 5, **caractérisé en ce que**
(a) des polyorganosiloxanes contenant des unités de formule générale
R⁴ₐ (R³O)_{b}SiO_{(4-a-b)/2} (IV)
dans laquelle
les R⁴ peuvent être identiques ou différents, et signifient un radical hydrocarboné monovalent, éventuellement substitué, de 1 à 30 atomes de carbone, ou un atome d'hydrogène,
les R³ peuvent être identiques ou différents, et signifient un atome d'hydrogène ou un radical hydrocarboné monovalent, éventuellement substitué,
a représente 0, 1, 2 ou 3, et
b représente 0, 1, 2 ou 3,
à condition que la somme a+b soit inférieure ou égale à 3 et que les polyorganosiloxanes contiennent 5 à 500 unités de formule (IV), b étant différent de 0 dans au moins une unité,
(b) un émulsifiant de formule (I), dont les groupes OH peuvent éventuellement être neutralisés en partie,
(c) un émulsifiant choisi dans le groupe constitué par les triglycérides éthoxylés contenant 40 à 400 groupes éthylène glycol, les esters de sorbitane d'acides gras éthoxylés contenant 12 à 18 atomes de carbone et 10 à 40 groupes éthylène glycol, les composés de formule (II) et les composés de formule (III),
(d) de l'eau et
éventuellement
(e) des substances supplémentaires
sont mélangés par agitation et/ou homogénéisation, et les organopolysiloxanes (a) contenant des unités de formule (IV) sont laissés se condenser à des températures de 0 à 50 °C jusqu'à ce que la viscosité souhaitée soit obtenue, puis l'émulsifiant de formule (I) est éventuellement neutralisé avec des bases, de sorte que le pH de l'émulsion soit supérieur à 5, et de l'eau (d) supplémentaire et/ou des substances (e) supplémentaires sont éventuellement ajoutées.

7. Procédé selon la revendication 6, **caractérisé en ce que** le composant (c) est utilisé en quantités de 1 à 25 parties en poids, par rapport à 100 parties en poids de polyorganosiloxanes (a).

8. Procédé selon la revendication 6 ou 7, **caractérisé en ce que**
lors d'une étape 1,
(a) 100 parties en poids de polyorganosiloxanes contenant des unités de formule générale
R⁴ₐ(R³O)_{b}SiO_{(4-a-b)/2} (IV)
dans laquelle
les R⁴ peuvent être identiques ou différents, et signifient un radical hydrocarboné monovalent, éventuellement substitué, de 1 à 30 atomes de carbone, ou un atome d'hydrogène,
les R³ peuvent être identiques ou différents, et signifient un atome d'hydrogène ou un radical hydrocarboné monovalent, éventuellement substitué,
a représente 0, 1, 2 ou 3, et
b représente 0, 1, 2 ou 3,
à condition que la somme a+b soit inférieure ou égale à 3 et que les organopolysiloxanes contiennent 5 à 500 unités de formule (IV), b étant différent de 0 dans au moins une unité,
(b) 1 à 30 parties en poids d'un émulsifiant de formule (I), dont les groupes OH peuvent éventuellement être neutralisés en partie,
(c) 1 à 30 parties en poids d'un émulsifiant choisi dans le groupe constitué par les triglycérides éthoxylés contenant 40 à 400 groupes éthylène glycol, les esters de sorbitane d'acides gras éthoxylés contenant 12 à 18 atomes de carbone et 10 à 40 groupes éthylène glycol, les composés de formule (II) et les composés de formule (III),
(d) 1 à 50 parties en poids d'eau et
éventuellement
(e) des substances supplémentaires
sont mélangés par agitation et/ou homogénéisation,
lors d'une étape 2 éventuellement réalisée,
de l'eau (d) supplémentaire est ajoutée,
lors d'une étape 3,
les organopolysiloxanes (a) contenant des unités de formule (IV) sont laissés se condenser à des températures de 0 à 50 °C jusqu'à ce que la viscosité souhaitée soit obtenue,
lors d'une étape 4 éventuellement réalisée, l'émulsifiant de formule (I) est neutralisé avec des bases, de sorte que le pH de l'émulsion soit supérieur à 5, et
lors d'une étape 5 éventuellement réalisée,
l'émulsion obtenue à l'étape 4 est mélangée avec de l'eau (d) supplémentaire et/ou des substances (e) supplémentaires.

9. Agent de soin pour le corps contenant des émulsions selon une ou plusieurs des revendications 1 à 5 et/ou fabriquées selon une ou plusieurs des revendications 6 à 8 en quantités de 0,05 à 10 % en poids.

10. Agent de soin pour le corps selon la revendication 9, **caractérisé en ce qu'**il s'agit d'un agent de soin pour les cheveux et **en ce qu'**il contient au moins un conditionneur.
